# EUROPEAN PATENT SPECIFICATION

(11) **EP 2 707 001 B1**
(45) Date of publication and mention of the grant of the patent: **21.12.2016**
(21) Application number: 12719756.4
(22) Date of filing: 11.05.2012
(51) Int. Cl.: A61K 31/519, A61P 35/00

(54) **METHOD FOR TREATMENT OF SOLID MALIGNANCIES INCLUDING ADVANCED OR METASTATIC SOLID MALIGNANCIES**
VERFAHREN ZUR BEHANDLUNG VON FESTEN MALIGNOMEN EINSCHLIESSLICH FORTGESCHRITTENER ODER METASTATISCHER FESTER MALIGNOME
PROCÉDÉ DE TRAITEMENT DE TUMEURS MALIGNES SOLIDES, Y COMPRIS DE TUMEURS MALIGNES SOLIDES MÉTASTASIQUES OU AVANCÉES

(30) Priority: 13.05.2011 EP 11165958
(43) Date of publication of application: 19.03.2014
(73) Proprietor: Boehringer Ingelheim International GmbH, 55216 Ingelheim am Rhein (DE)
(72) Inventor: TAUBE, Tillmann, 55216 Ingelheim Am Rhein (DE); MUNZERT, Gerd Michael, 55216 Ingelheim Am Rhein (DE); RUDOLPH, Dorothea, 55216 Ingelheim Am Rhein (DE)
(74) Representative: Simon, Elke Anna Maria
(86) International application number: PCT/EP2012/058704
(87) International publication number: WO 2012/156283

(56) References cited:
- SCHÖFFSKI, P.: "Polo-like kinase (PLK) inhibitors in preclinical and early clinical development in oncology", THE ONCOLOGIST, vol. 14, 2009, pages 559-570, XP009161141,
- SCHÖFFSKI ,P. ET AL.: "A phase I single dose escalation study of the novel Polo-like kinase 1 inhibitor BI 6727 in patients with advanced solid tumours", EJC SUPPLEMENT, vol. 6, no. 12, October 2008 (2008-10), pages 14-15, XP002679932,
- Anonymous: "BI 6727 (volasertib) monotherapy phase I trial in Japanese patients with advanced solid tumours", ClinicalTrials.gov, 29 April 2011 (2011-04-29), XP002679933, Retrieved from the Internet: URL:http://www.clinicaltrials.gov/ct2/show /NCT01348347?term=volasertib&rank=1 [retrieved on 2012-07-16]

## Description

The present invention relates to the use of Volasertib or a salt thereof or a hydrate thereof for treating patients suffering from solid malignancies including advanced or metastatic solid malignancies comprising a high frequency administration of Volasertib according to a specific dosage schedule.

### Background of the invention

Most advanced or metastatic human cancers are incurable despite the availability of a variety of established treatment modalities like surgery, cytotoxic drugs, radiation therapy, and combinations of these. Objective responses in patients with advanced disease, though frequently seen using these treatments, are often followed by tumour progression and death. Therefore the search for new therapeutic strategies has become an urgent priority.

The efficacy of chemotherapeutic agents can be improved by improving the dosage schedule. Even if the concept of improved dosage schedules already has been suggested, there is still a need for new and efficient therapeutic concepts for the treatment of cancer diseases, which show advantages over standard therapies.

Volasertib is a highly potent and selective inhibitor of the serine-threonine Polo like kinase 1 (Plk1), a key regulator of cell-cycle progression. Volsaertib is a dihydropteridinone derivative with distinct pharmacokinetic (PK) properties. Schöffski et al. described already the treatment of solid tumors and escalation studies of volasertib in patients with advanced solid tumors (The Oncologist 2009, Vol. 14, pp. 559 - 570; EJC Supplement 2008; Vol. 6, No. 12, pp. 14 - 15). The problem underlying this invention was to develop improved dosage schedules for monotherapy of advanced or metastatic solid malignacies.

Volasertib (I) is known as the compound N-[trans-4-[4-(cyclopropylmethyl)-1-piperazinyl]cyclohexyl]-4-[[(7R)-7-ethyl-5,6,7,8-tetrahydro-5-methyl-8-(1-methylethyl)-6-oxo-2-pteridinyl]amino]-3-methoxy-benzamide,

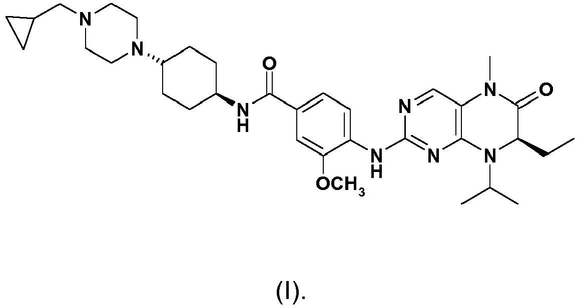

This compound is disclosed in WO 04/076454. Furthermore, trihydrochloride salt forms and hydrates thereof are known from WO 07/090844. They possess properties which make those forms especially suitable for pharmaceutical use. The above mentioned patent applications further disclose the use of this compound or its monoethanesulfonate salt for the preparation of pharmaceutical compositions intended especially for the treatment of diseases characterized by excessive or abnormal cell proliferation.

### Summary of the Invention

According to the state of the art Volasertib is administered once in a 21 day treatment cycle if applied as monotherapy in treatment of solid malignancies including advanced or metastatic solid malignancies. It has now been found that Volasertib can be administered in shorter intervals than so far used.

Therefore, a first object of the present invention refers to Volasertib or a pharmaceutically acceptable salt thereof or a hydrate thereof for the use in treating patients suffering from solid malignancies including advanced or metastatic solid malignancies characterized by administration of 50 to 200 mg, preferably 150 mg of Volasertib or a pharmaceutically acceptable salt thereof or a hydrate thereof at two days within a 21 day treatment cycle. Preferably Volasertib is administered at day 1 and at one of the days 5, 6, 7, 8, 9, 10, 11 or 12 more preferably at day 1 and at day 8 of the 21 day treatment cycle. Preferably equal doses of Volasertib are administered at each of the above mentioned two days during the 21 day treatment cycle.

Further disclosed is a pharmaceutical composition comprising an effective amount of Volasertib or a pharmaceutically acceptable salt thereof or a hydrate thereof together with an instruction for administration of Volasertib to a patient suffering from solid malignancies including advanced or metastatic solid malignancies, wherein according to said instruction 50 to 200 mg Volasertib or a pharmaceutically acceptable salt thereof or a hydrate thereof are to be administered at two days during a 21 day treatment cycle.

Further disclosed is a pharmaceutical composition comprising an effective amount of Volasertib or a pharmaceutically acceptable salt thereof or a hydrate thereof together with an instruction for administration of Volasertib to a patient suffering from solid malignancies including advanced or metastatic solid malignancies, wherein according to said instruction 50 to 200 mg Volasertib or a pharmaceutically acceptable salt thereof or a hydrate thereof are to be administered at day 1 and at one of the days 5, 6, 7, 8, 9, 10, 11 or 12 during a 21 day treatment cycle.

Further disclosed is a pharmaceutical composition comprising an effective amount of Volasertib or a pharmaceutically acceptable salt thereof or a hydrate thereof together with an instruction for administration of Volasertib to a patient suffering from solid malignancies including advanced or metastatic solid malignancies, wherein according to said instruction 50 to 200 mg Volasertib or a pharmaceutically acceptable salt thereof or a hydrate thereof are to be administered at day 1 and at day 8 during a 21 day treatment cycle.

Further disclosed is a pharmaceutical composition according to any one of the compositions above wherein according to said instruction 150 mg Volasertib or a pharmaceutially acceptable salt thereof or a hydrate thereof are to be administered.

### Detailed Description of the Invention

For example, the administration of Volasertib at two days within a 21 day treatment cycle means that Volasertib is administered at two different days during a 21 day treatment cycle.

The administration of Volasertib at day 1 and 8 during a 21 day treatment cycle means that one dosage of Volasertib or a pharmaceutically acceptable salt or a hydrate thereof is administered at day 1 and the second dosage is administered at day 8 of the 21 day treatment cycle to the patient suffering from solid malignancies including advanced or metastatic solid malignancies.

Accordingly a complete 21 day treatment cycle according to one of the above mentioned dosage schedules may comprise the following administrations:
Day 1: one dosage of Volasertib (e.g. 150 mg);
Day 8: one dosage of Volasertib (e.g. 150 mg);
Day 2 to 7 and day 9 to 21 (including): no administration of Volasertib.

This treatment cycle can be repeated as long as patients are eligible for repeated cycles, i.e. until progression of disease, or unacceptable toxicity and as long as neither patient nor investigator requests treatment discontinuation.

The instruction for administration may be in any form suitable for pharmaceuticals, e.g. in form of a leaflet added to the dosage form within secondary packaging or an imprint on the primary or secondary packaging.

### Dosages / Volasertib:

For intraveneous treatment Volasertib may be administered to the human patient in a daily dose of 50 to 200 mg/application, preferably 150 mg/application. For instance, Volasertib can be administered as a slow intravenous infusion over several hours, e.g. over about 1, 2, 4, 6, 10, 12 or 24 hours, preferably about 1 or 2 hours.

However, it may optionally be necessary to deviate from the dosage amounts specified for Volasertib, depending on the body weight or method of administration, the individual response to the medication, the nature of the formulation used and the time or interval over which it is administered. Thus, in some cases, it may be sufficient to use less than the minimum quantity specified above, while in other cases the upper limit specified will have to be exceeded. When large amounts are administered it may be advisable to spread them over the day in a number of single doses.

### Dosage Forms and Formulation Aspects

Regarding any aspects of the invention for Volasertib pharmaceutically acceptable salts or hydrates thereof may be used, preferably trihydrochloride salt forms and hydrates thereof as disclosed in WO 07/090844. Dosages or amounts of the actives provided in the context of this invention refer in any case to the free base equivalent, that is Volasertib in the free base form.

The term "therapeutically effective amount" shall mean that amount of a drug or pharmaceutical agent that will elicit the biological or medical response of a tissue system, animal or human that is being sought by a researcher or clinician, resulting in a beneficial effect for at least a statistically significant fraction of patients, such as a improvement of symptoms, a cure, a reduction in disease load, reduction in tumor mass, extension of life, or improvement in quality of life.

Day 1 of a 21 day treatment cycle is defined as that day at which the first dose of Volasertib is administered.

The term "advanced or metastatic solid malignancies" is defined as histologically or cytologically confirmed diagnosis of advanced, non resectable and/or metastatic relapsed or refractory solid malignant tumor, not amenable to standard therapy or for which no therapy of proven efficacy exists. Solid malignant tumors according to the present invention comprise but are not limited to carcinomas, sarcomas, melanomas, and lymphomas.

Examples of carcinomas within the scope of the invention include but are not limited to adenocarcinoma (AC), squamous cell carcinoma (SCC) and mixed or undifferentiated carcinomas. Carcinomas within the scope of the invention include but are not limited to the following histologies:
- Head and neck tumours: SCC, AC, transitional cell cancers, mucoepidermoid cancers, undifferentiated carcinomas;
- Central nervous system tumours: Astrocytoma, glioblastoma, meningeoma, neurinoma, schwannoma, ependymoma, hypophysoma, oligodendroglioma, medulloblastoma;
- Bronchial and mediastinal tumours:
   ∘ Bronchial tumours:
      ▪ Small cell lung cancers (SCLC): oat-cell lung cancer, intermediate cell cancer, combined oat-cell lung cancer;
      ▪ Non-small cell lung cancers (NSCLC): SCC, spindle cell carcinoma, AC, bronchioalveolar carcinoma, large cell NSCLC, clear cell NSCLC;
   ∘ Mesothelioma;
   ∘ Thymoma;
   ∘ Thyroid carcinomas: papillary, follicular, anaplastic, medullary;
- Tumours of the gastrointestinal tract:
   ∘ Oesophageal cancers: SCC, AC, anaplastic, carcinoid, sarcoma;
   ∘ Gastric cancers: AC, adenosquamous, anaplastic;
   ∘ Colorectal cancers: AC, including hereditary forms of AC, carcinoid, sarcoma;
   ∘ Anal cancers: SCC, transitional epithelial cancer, AC, basal cell carcinoma;
   ∘ Pancreatic cancers: AC, including ductal and acinary cancers, papillary, adenosquamous, undifferentiated, tumours of the endocrine pancreas;
   ∘ Hepatocellular carcinoma, cholangiocarcinoma, angiosarcoma, hepatoblastoma;
   ∘ Biliary carcinomas: AC, SCC, small cell, undifferentiated;
   ∘ Gastrointestinal stroma tumours (GIST);
- Gynaecological cancers:
   ∘ Breast cancers: AC, including invasive ductal, lobular and medullary cancers, tubular, mucinous cancers, Paget-carcinoma, inflammatory carcinoma, ductal and lobular carcinoma in situ;
   ∘ Ovarian cancers: Epithelial tumours, stroma tumours, germ cell tumours, undifferentiated tumours;
   ∘ Cervical cancers: SCC, AC, mixed and undifferentiated tumours;
   ∘ Endometrial cancers: AC, SCC, mixed, undifferentiated tumours;
   ∘ Vulvar cancers: SCC, AC;
   ∘ Vaginal cancers: SCC, AC;
- Urinary tract and testicular cancers:
   ∘ Testicular cancers: seminoma;
   ∘ Non-seminomatous germ cell tumours: teratoma, embryonal cell carcinoma, choriocarcinoma, yolk sac tumour, mixed, Sertoli and Leydig-cell tumours;
   ∘ Extragonadal germ cell tumours;
   ∘ Prostate cancers: AC, small cell, SCC;
   ∘ Renal cell cancers: AC, including clear cell, papillary and chromophobous carcinomas, hereditary forms (e.g. von-Hippel-Lindau syndrome), nephroblastoma;
   ∘ Urinary bladder cancers: transitional cell (urothelial) cancers, SCC, AC;
   ∘ Urethral cancers: SCC, transitional cell cancers, AC;
   ∘ Penile cancers: SCC;
- Tumours of endocrine tissue:
   ∘ Thyroid cancers: papillary, follicular, anaplastic, medullary carcinomas, including MEN syndrome;
   ∘ Tumours of the endocrine pancreas;
   ∘ Carcinoids;
   ∘ Pheochromocytoma.

Examples of sarcomas within the scope of the invention include but are not limited to Ewing-sarcoma, osteosarcoma or osteogenic sarcoma, chondrosarcoma, synovial sarcoma, leiomyosarcoma, rhabdomyosarcoma, mesothelial sarcoma or mesothelioma, fibrosarcoma, angiosarcoma or hemangioendothelioma, liposarcoma, glioma or astrocytoma, myxosarcoma, malignant fibrous histiocytoma, mesenchymous or mixed mesodermal tumour, neuroblastoma and clear cell sarcoma.

Examples of melanomas within the scope of the invention include but are not limited to superficial spreading melanoma, nodular and lentigo-maligna melanoma.

Examples of lymphomas within the scope of the invention include but are not limited to:
- Hodgkin-lymphoma;
- Non-Hodgkin-lymphomas: T- and B-cell lymphomas
   ∘ B-celllymphomas:
      ▪ Low and intermediate grade: Chronic lymphocytic leukemia (CLL), prolymphocytic leukemia (PLL), small lymphocytic lymphoma, hairy cell leukemia, plasmacytoid lymphoma, mantle cell lymphoma, follicular lymphoma, marginal zone lymphoma including MALT-lymphoma;
      ▪ High grade: diffuse large B-cell lymphoma (DLBCL including immunoblastic and centroblastic variants), lymphoblastic, Burkitt's lymphoma;
   ∘ T-cell lymphomas:
      ▪ Low grade: T-CLL, T-PLL, Mycosis fungoides, Sezary-syndrome;
      ▪ High grade: Anaplastic large cell, T-immunoblastic and lymphoblastic.

In accordance with the present invention Volasertib may be administered by parenteral (e.g. intramuscular, intraperitoneal, intravenous, transdermal or subcutaneous injection), preferably intravenous application, and may be formulated, alone or together, in suitable dosage unit formulations containing conventional nontoxic pharmaceutically acceptable carriers, adjuvants and vehicles appropriate for each route of administration. Dosage forms and formulations of both actives suitable within the present invention are known in the art. For instance, such dosage forms and formulations include those disclosed for Volasertib in WO 2006/018221.

The following Examples serve to illustrate the invention without restricting it:

### Example 2: Clinical Trial

**Material and Methods:** Sequential cohorts of 3-6 patients with advanced or metastatic solid malignancies received a 2-hr infusion of volasertib on days 1 and 8 every 3 weeks in a toxicity guided dose escalation study. There were 4 prespecified doses (50-200 mg).
**Results**: 27 Asian patients with advanced solid malignancies were treated. To date, reversible thrombocytopenia, neutropenia and febrile neutropenia were dose limiting toxicities (DLTs). Fatigue, decreased appetite, and nausea were among the most frequent drug-related non-hematologic events. MTD was 150 mg for above described schedule. The median number of initiated courses was 3 over all dose groups (range 1-21). Volasertib exhibited multi-compartmental PK behavior with a mean terminal elimination half-life of 107 hours, moderate clearance (807 mL/min) and a large volume of distribution (4500 L). Two patients with bladder cancer and melanoma, respectively had partial responses. These results demonstrate preliminary anti-tumor activity (1 patient achieved partial response, 10 patients achieved stable disease) if Volasertib is administered at two days during a 21 day treatment cycle.

## Claims

1. Volasertib or a pharmaceutically acceptable salt thereof or a hydrate thereof for the use in treating solid malignancies including advanced or metastatic solid malignancies, **characterized by** administration of 50 to 200 mg Volasertib or a pharmaceutically acceptable salt thereof or a hydrate thereof at two days during a 21 day treatment cycle.

2. Volasertib or a pharmaceutically acceptable salt thereof or a hydrate thereof for the use according to claim 1, wherein Volasertib or a pharmaceutically acceptable salt thereof or a hydrate thereof is administered at day 1 and at one of the days 5, 6, 7, 8, 9, 10, 11 or 12 during a 21 day treatment cycle.

3. Volasertib or a pharmaceutically acceptable salt thereof or a hydrate thereof for the use according to claim 1 or 2, wherein Volasertib or a pharmaceutically acceptable salt thereof or a hydrate thereof is administered at day 1 and at day 8 during a 21 day treatment cycle.

4. Volasertib or a pharmaceutically acceptable salt thereof or a hydrate thereof for the use according to any one of the claims 1 to 3, wherein 150 mg of Volasertib or a pharmaceutically acceptable salt thereof or a hydrate thereof are administered per day of administration.

## Patentansprüche

1. Volasertib oder ein pharmazeutisch akzeptables bzw. annehmbares Salz hiervon oder ein Hydrat hiervon zur Verwendung in der Behandlung solider Malignome, einschließlich fortgeschrittener oder metastasierender solider Malignome, **gekennzeichnet durch** die Verabreichung von 50 bis 200 mg Volasertib oder eines pharmazeutisch akzeptablen bzw. annehmbaren Salzes hiervon oder eines Hydrates hiervon an zwei Tagen während eines 21-tägigen Behandlungszyklus.

2. Volasertib oder ein pharmazeutisch akzeptables bzw. annehmbares Salz hiervon oder ein Hydrat hiervon zur Verwendung nach Anspruch 1, wobei Volasertib oder ein pharmazeutisch akzeptables bzw. annehmbares Salz hiervon oder ein Hydrat hiervon an Tag 1 und an einem der Tage 5, 6, 7, 8, 9, 10, 11 oder 12 während eines 21-tägigen Behandlungszyklus verabreicht wird.

3. Volasertib oder ein pharmazeutisch akzeptables bzw. annehmbares Salz hiervon oder ein Hydrat hiervon zur Verwendung nach Anspruch 1 oder 2, wobei Volasertib oder ein pharmazeutisch akzeptables bzw. annehmbares Salz hiervon oder ein Hydrat hiervon an Tag 1 und an Tag 8 während eines 21-tägigen Behandlungszyklus verabreicht wird.

4. Volasertib oder ein pharmazeutisch akzeptables bzw. annehmbares Salz hiervon oder ein Hydrat hiervon zur Verwendung nach irgendeinem der Ansprüche 1 bis 3, wobei 150 mg Volasertib oder ein pharmazeutisch akzeptables bzw. annehmbares Salz hiervon oder ein Hydrat hiervon pro Verabreichungstag verabreicht werden.

## Revendications

1. Volasertib ou l'un de ses sels pharmaceutiquement acceptables ou l'un de ses hydrates pour son utilisation dans le traitement d'affections malignes solides y compris des affections malignes solides à un stade avancé ou métastasées, **caractérisé par** l'administration de 50 à 200 mg de Volasertib ou de l'un de ses sels pharmaceutiquement acceptables ou de l'un de ses hydrates deux jours durant un cycle de traitement de 21 jours.

2. Volasertib ou l'un de ses sels pharmaceutiquement acceptables ou l'un de ses hydrates pour son utilisation selon la revendication 1, le Volasertib ou l'un de ses sels pharmaceutiquement acceptables ou l'un de ses hydrates étant administré au jour 1 et à l'un des jours 5, 6, 7, 8, 9, 10, 11 ou 12 durant un cycle de traitement de 21 jours.

3. Volasertib ou l'un de ses sels pharmaceutiquement acceptables ou l'un de ses hydrates pour son utilisation selon la revendication 1 ou 2, le Volasertib ou l'un de ses sels pharmaceutiquement acceptables ou l'un de ses hydrates étant administré au jour 1 et au jour 8 durant un cycle de traitement de 21 jours.

4. Volasertib ou l'un de ses sels pharmaceutiquement acceptables ou l'un de ses hydrates pour son utilisation selon l'une quelconque des revendications 1 à 3, 150 mg de Volasertib ou de l'un de ses sels pharmaceutiquement acceptables ou de l'un de ses hydrates étant administrés par jour d'administration.
